# EUROPEAN PATENT SPECIFICATION

(11) **EP 1 902 661 B1**
(45) Date of publication and mention of the grant of the patent: **30.12.2015**
(21) Application number: 06768215.3
(22) Date of filing: 14.07.2006
(51) Int. Cl.: A61B 1/00

(54) **ENDOSCOPE DEVICE**
ENDOSKOPVORRICHTUNG
DISPOSITIF ENDOSCOPE

(30) Priority: 14.07.2005 JP 2005205802
(43) Date of publication of application: 26.03.2008
(73) Proprietor: Olympus Corporation, Shibuya-ku Tokyo (JP)
(72) Inventor: NOGUCHI, Toshiaki c/o Intellectual Property Support Department, Hachioji-shi Tokyo 192-8512 (JP); TANIGUCHI, Akira, 20253 Hamburg (DE); KOITABASHI, Masanobu c/o Intellectual Property Support Department, Hachioji-shi Tokyo 192-8512 (JP); NAKAMOTO, Kouji, Tokyo 193-0933 (JP)
(74) Representative: von Hellfeld, Axel
(86) International application number: PCT/JP2006/314055
(87) International publication number: WO 2007/007875

(56) References cited:
- EP-A2- 0 689 793
- JP-A- 04 259 440
- JP-A- 09 187 414
- JP-A- 11 123 174
- JP-A- 2000 023 910
- US-A- 6 095 971
- US-A1- 2003 216 617

## Description

### Technical Field

The present invention relates to an endoscope apparatus which is inserted in a lumen that is an object of an inspection, and is used to observe an inside of, e.g. a living body organ, and to perform therapeutic treatment for a part that undergoes an operation.

### Background Art

In general, in an endoscope apparatus, an elongated soft endoscope insertion section extends from an endoscope body. This endoscope insertion section is inserted into a lumen that is an object of an inspection, such as an organ or a body cavity of a patient, and a diseased part is observed. Alternatively, the insertion section, which has been inserted in the lumen that is the object of the inspection, is combined with a therapeutic device at a time of use. Thus, the endoscope apparatus is used for therapeutic treatment of a diseased part in the body, such as resection of a mucous membrane, without ventrotomy.

In this endoscope apparatus, a bend section with a soft distal end, which is capable of performing a bend operation, is provided at a distal end portion of the endoscope insertion section. With this structure, the insertion of the insertion section into a lumen that is the object of an inspection, e.g. an organ, is facilitated, and the inside of the object of the inspection can entirely be observed. Within the endoscope insertion section, various conduits, such as an insertion channel for the therapeutic device, an air feed conduit, a liquid feed conduit and a suction conduit, are provided (see, e.g. Jpn. Pat. Appln. KOKAI Publication No. H6-54795 and Jpn. Pat. Appln. KOKAI Publication No. 2000-107123).

In the meantime, as regards the endoscope apparatus, a high-level insertion operation is required at the time of a work of inserting the insertion section into a desired position within the lumen that is the object of the inspection. For example, an operation of advancing/retreating the insertion section in the direction of insertion is needed in combination with an operation of bending the distal end portion of the insertion section or turning the insertion section about its axis.

In particular, in the case of performing a work of inserting the insertion section of the endoscope into the large intestine, the work of inserting the insertion section is difficult. In this case, the large intestine itself has a complex shape, and the shape of the large intestine greatly varies from patient to patient. Thus, at the time of performing the work of inserting the insertion section of the endoscope, air is fed via an air feed mechanism into the air feed conduit within the endoscope insertion section, and the work of inserting the endoscope insertion section is conducted while air is being fed into the large intestine through the air feed conduit. Thereafter, the therapeutic device, such as a forceps, is inserted into the therapeutic device insertion channel of the endoscope body. At this time, while water is being fed into the liquid feed conduit within the endoscope insertion section, therapeutic treatment is performed for a part that undergoes treatment. Further, an object to be sucked, such as removed matter after treatment, is sucked through the suction conduit within the endoscope insertion section and is discharged to the outside.

As described above, when the work of inserting the insertion section of the endoscope into the large intestine, various operations, such as adjustment of amounts of air and water that are fed into the organ, and adjustment of an amount of suction from the organ, are necessary. Thus, high-level adjustment works are required for the operations of air feed, water feed and suction, and the difficulty of these operations is high. As a result, in the case of giving instruction to less experienced surgeons as to how to insert the insertion section of the endoscope into the large intestine, such a method has conventionally been adopted that the actual insertion operation of the insertion section of the endoscope is observed under the tuition of well experienced doctors, and the know-how of works of, e.g. the adjustment of the water feed, water feed and suction operations is learned.

US 6,095,971 discloses an endoscope apparatus having an operation section comprising switches for controlling an operation of the operation section and an auxiliary operation section comprising a second set of switches for controlling an operation of the operation section. A permission switch disposed on the operation, section makes operation of the second set of switches of the auxiliary operation section effective such that the operation section can be operated remotely from the auxiliary operation section when the endoscope is used as a laparoscope or when the endoscope is cleaned.

### Disclosure of Invention

In general, in the case of making less experienced surgeons learn the know-how of the work of inserting the insertion section of the endoscope into the large intestine, the surgeons, who learn therapy techniques, conventionally acquire such techniques only visually by observing the actual procedures of insertion, which are performed by well experienced doctors. Hence, there is the problem that a great deal of time is needed until less experienced surgeons learn the high-level therapy technique of inserting the insertion section of the endoscope into the large intestine.

The present invention has been made in consideration of the above-described circumstances, and the object of the invention is to provide an endoscope apparatus which can realize simple and easy operations of air feed, water feed and suction, which are necessary when a high-level insertion technique is performed, and can improve handling operability.

According to a first aspect of the present invention, an endoscope apparatus comprising: an endoscope including an insertion section which is inserted in a lumen that is an object of an inspection, and an operation section having an operation element which performs an operation of the insertion section; detection means for detecting an operation state of the operation element; and a separate operation section which is disposed separately from the endoscope, wherein the separate operation section includes a display section which displays the operation state of the operation element, which is detected by the detection means, and an operation switch which operates and controls the operation element.

Preferably, the insertion section includes an air feed conduit, a water feed conduit and a suction conduit, the operation element includes: an air feed mechanism which feeds air to the air feed conduit and feeds the air into the object of the inspection; a water feed mechanism which feeds water to the water feed conduit and feeds the water into the object of the inspection; and a suction mechanism which sucks, via the suction conduit, matter to be sucked in the object of the inspection, the detection means detects an air feed amount of the air that is fed from the air feed mechanism, a water feed amount of the water that is fed from the water feed mechanism, and a suction amount of the matter sucked by the suction mechanism, and the separate operation section includes a display section which displays the air feed amount, the water feed amount and the section amount which are detected by the detection means, and operation switches which operate and control the air feed mechanism, the water feed mechanism and the suction mechanism.

According to the above-described structure, if the insertion section is inserted into the object of the invention and the air feed mechanism, water feed mechanism and suction mechanism are driven and controlled to execute air feed, water feed and suction, the air feed amount, water feed amount and suction amount are detected by the detection means and displayed on the display section of the separate operation section. By visually recognizing the air feed amount, water feed amount and suction amount that are displayed on the display section, the current state can be understood. At this time, by selectively operating the operation switches of the separate operation section on the basis of the data displayed on the display section, the air feed mechanism, water feed mechanism and suction mechanism can be operated and controlled. Therefore, it is possible to provide an endoscope apparatus which can realize simple and easy operations of air feed, water feed and suction, which are necessary when a high-level insertion technique is performed, and can improve handling operability.

The separate operation section includes switching means for effecting selective switching between an operation of the operation element and an operation of the operation switches, and one of the operation of the operation element and the operation of the operation switches is selectable.

Preferably, the separate operation section includes alarm means for giving an alarm when the air feed amount, the water feed amount and the suction amount, which are detected by the detection means, have reached arbitrary preset values.

Preferably, the alarm means flickers display of the air feed amount, the water feed amount and the suction amount on the display section.

Preferably, the alarm means produces an alarm sound.

Preferably, the separate operation section is wirelessly connected to the detection means.

### Brief Description of Drawings

FIG. 1 schematically shows the structure of the entire system of an endoscope apparatus according to a first embodiment of the present invention;
FIG. 2A is a schematic cross-sectional structural view showing a state in which a connection end of an endoscope insertion section and an operation section-side connection portion are disconnected in the endoscope apparatus according to the first embodiment;
FIG. 2B is a schematic structural view showing an endoscope control device of the endoscope apparatus according to the first embodiment; and
FIG. 3 is a schematic cross-sectional structural view showing a state in which a separate operation section is connected to the endoscope control device according to the first embodiment.

### Best Mode for Carrying Out the Invention

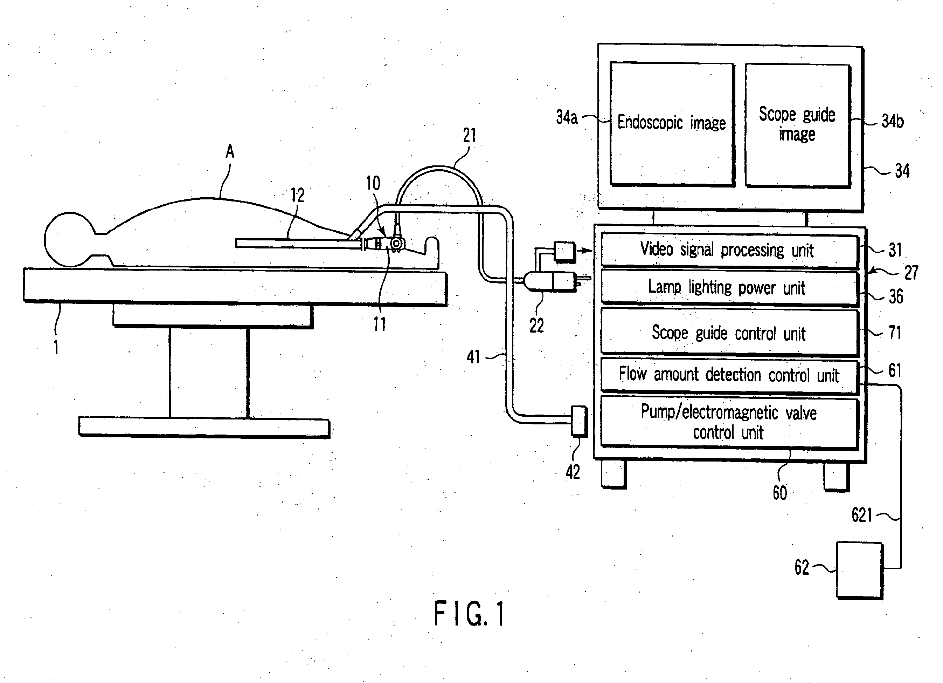

A first embodiment of the present invention will now be described with reference to FIG. 1 to FIG. 3. FIG. 1 schematically shows the structure of the entire system of an endoscope apparatus according to the first embodiment of the invention. The system of the endoscope apparatus includes an electronic endoscope 10 and an endoscope control device 27.

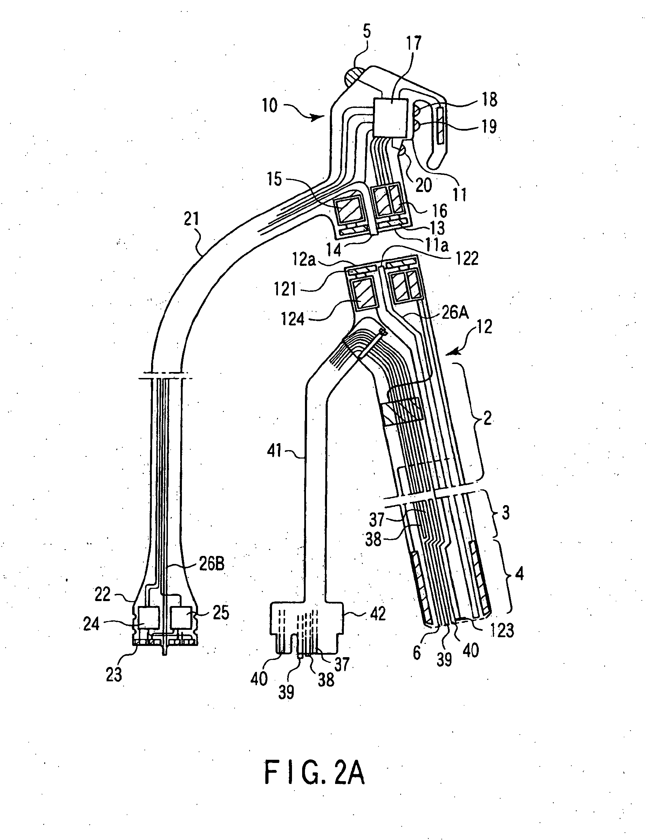

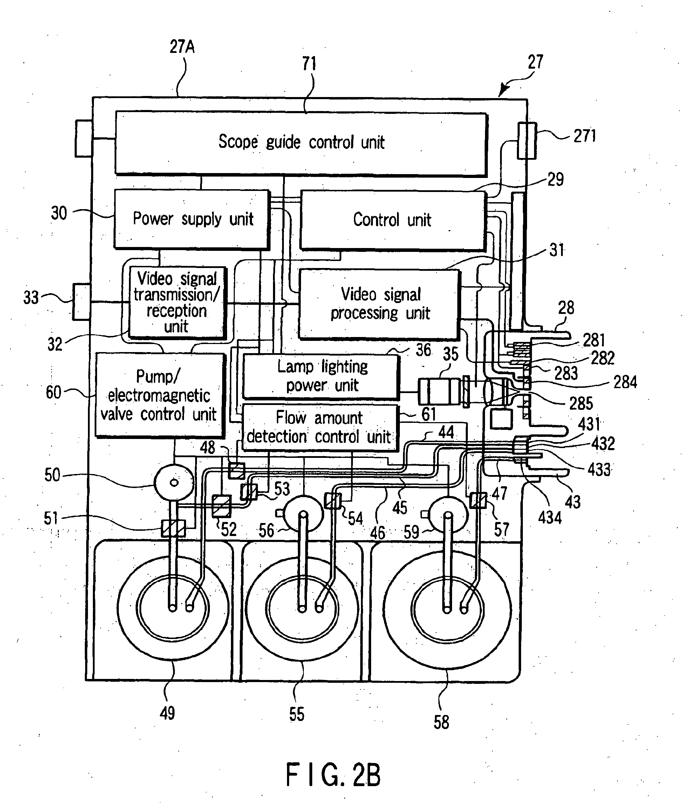

As shown in FIG. 2A, the electronic endoscope 10 includes an elongated soft insertion section 12 and an operation section 11 which is coupled to a proximal end portion of the insertion section 12. The insertion section 12 is inserted in a lumen of an object of an inspection, for example, in a body cavity of a patient A lying on a bed 1. In the present embodiment, the operation section 11 and the insertion section 12 are detachably coupled.

The operation section 11 is provided with an operation-section-side transmission/reception coil 13 and a light guide connection portion 14 at an attachment/detachment section 11a for attachment/detachment to/from the insertion section 12. The insertion section 12 is provided with an insertion-section-side transmission/reception coil 121 and a light guide connection portion 122 at a connection end (attachment/detachment section) 12a for attachment/detachment to/from the operation section 11. When the attachment/detachment section 11a of the operation section 11 and the connection end 12a of the insertion section 12 are coupled, the operation-section-side transmission/reception coil 13 and the insertion-section-side transmission/reception coil 121 are detachably connected and also the light guide connection portion 14 on the operation section 11 side and the light guide connection portion 122 on the insertion section 12 side are detachably connected. Thereby, the attachment/detachment section 11a of the operation section 11 and the connection end 12a of the insertion section 12 are electrically, optically and mechanically coupled.

The insertion section 12 includes an elongated flexible tube section 2, a bend section 3 which is coupled to a distal end of the flexible tube section 2, and a distal-end hard section 4 which is coupled to a distal end of the bend section 3. Further, one end portion of a conduit tube 41 is connected to a proximal end portion of the flexible tube section 2. A tube connector 42 is connected to the other end portion of the conduit tube 41.

A plurality of conduits and a light guide 26A are inserted in the insertion section 12. The plurality of conduits include a water feed conduit 37, an air feed conduit 38, a sub-water feed conduit 39 and a suction conduit 40. The water feed conduit 37, air feed conduit 38, sub-water feed conduit 39 and suction conduit 40 are made to extend within the conduit tube 41, and their extended end portions are fixed to the tube connector 42. The tube connector 42 of the conduit tube 41 is detachably connected to a conduit connector receiving section 43 of the endoscope control device 27 (to be described lager).

The distal-end hard section 4 is provided with an air/water feed nozzle 6, distal end opening portions of the sub-water feed conduit 39 and suction conduit 40, an observation lens (not shown), and an illumination lens. The air/water feed nozzle 6 is coupled to the water feed conduit 37 and air feed conduit 38. An imaging device 123, such as a CCD, is provided behind the observation lens. An emission end portion of the light guide 26A is provided behind the illumination lens. An incidence end portion of the light guide 26A is optically connected to the light guide connection portion 122 at the connection end 12a of the insertion section 12.

A proximal end portion of a universal cord 21 is coupled to the operation section 11. A scope connector 22 is coupled to a distal end portion of the universal cord 21. The scope connector 22 is provided with a cord-side transmission/reception coil 23, a power supply control circuit 24 and a video signal processing circuit 25. Further, the operation section 11 is provided with a bend operation device 5, an air feed operation switch 18, a water feed operation switch 19, a suction operation switch 20 and various operation buttons for imaging of the endoscope 10. By the operation of the bend operation section 11, the bend section 3 of the insertion section 12 is operated and the bending of the distal end portion of the insertion section 12 is adjusted.

A video signal processing circuit 15 and a driving system processing circuit 16 are connected to the operation-section-side transmission/reception coil 13. The video signal processing circuit 15 and driving system processing circuit 16 are connected to a control circuit 17. The air feed operation switch 18, water feed operation switch 19, a sub-water feed operation switch (not shown in FIG. 2A for a reason of drawing) and suction operation switch 20 are connected to the control circuit 17.

The control circuit 17 is connected to the cord-side transmission/reception coil 23, which is provided in the scope connector 22 of the universal cord 21, via the power supply control circuit 24 and video signal processing circuit 25. A light guide 26B is inserted in the universal cord 21. One end of the light guide 26B is optically connected to the light guide connection portion 14. The other end of the light guide 26B is connectably projected from the scope connector 22 of the universal cord 21. The scope connector 22 of the universal cord 21 is detachably attached to on a scope connector receiving section 28 of the endoscope control device 27 (to be described later).

The endoscope control device 27 includes a video signal processing unit 31, a lamp lighting power unit 36, a pump/electromagnetic valve control unit 60, a flow amount detection control unit 61, a scope guide control unit 71, and a display device 34 which is connected to the video signal processing unit 31. The pump/electromagnetic valve control unit 60 controls air feed, water feed and suction. The scope guide control unit 71 includes necessary-part pressing force amount detection. Further, the display device 34 includes a screen 34a which displays an endoscopic image, and a screen 34b which displays a scope guide image.

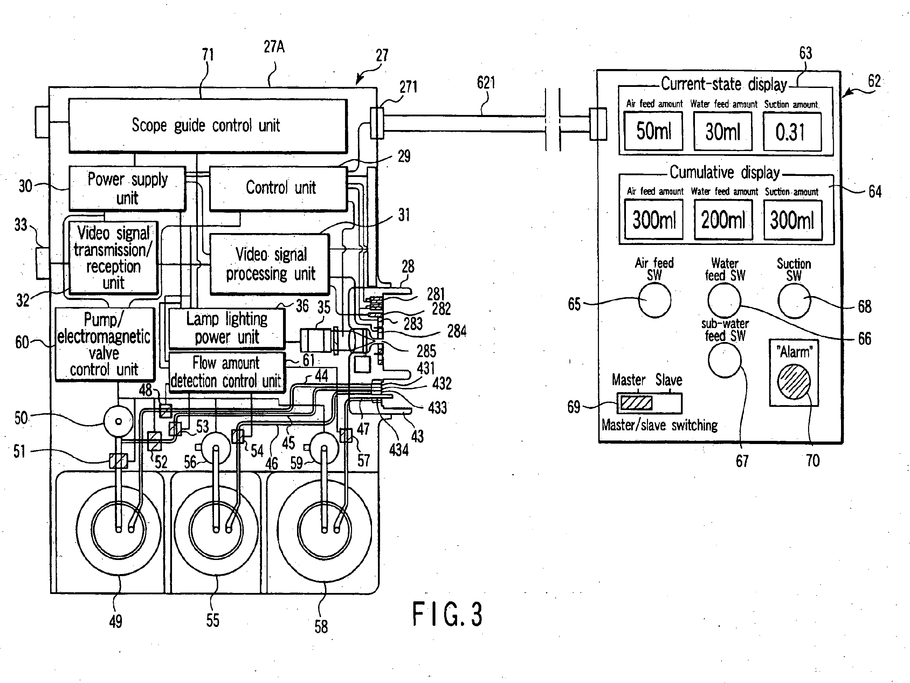

In addition, an outer casing 27A of the endoscope control device 27 is provided with a scope connector receiving section 28, a conduit connector receiving section 43, an external connection connector 271, and an antenna 33. The scope connector 22 of the electronic endoscope 10 is detachably coupled to the scope connector receiving section 28, and the tube connector 42 is detachably coupled to the conduit connector receiving section 43. A separate operation section 62 (see FIG. 3), which is to be described later, is connected to the external connection connector 271 via a cable 621 (see FIG. 3).

The scope connector receiving section 28 is provided with a power terminal 281, a video signal terminal 282, a first transmission transformer 283, a second transmission transformer 284, and a light guide connection terminal 285. When the scope connector receiving section 28 and the scope connector 22 of the universal cord 21 are connected, the light guide 26 of the scope connector 22 is detachably coupled to the light guide connection terminal 285 of the scope connector receiving section 28. Further, the transmission/reception coil 23 of the scope connector 22 is detachably coupled to the power terminal 281, video signal terminal 282, first transmission transformer 283 and second transmission transformer 284 of the scope connector receiving section 28.

A control unit 29, a power supply unit 30, a video signal transmission/reception unit 32, a water feed bottle 49, a sub-water feed bottle 55 and a suction bottle 58 are provided within the endoscope control device 27. The power terminal 281 of the scope connector receiving section 28 is connected to the power supply unit 30 via the control unit 29.

The video signal terminal 282 is connected to the antenna 33 via the video signal processing unit 31 and video signal transmission/reception unit 32. Image data, which is captured by the imaging device 123, which is disposed at the distal end portion of the insertion section 12, is guided to the transmission/reception coil 121 via a video signal processing circuit 124 that is provided in the insertion section 12. Subsequently, the image data is delivered from the transmission/reception coil 121 to the transmission/reception coil 13 of the operation section 11. The image data is input to the video signal terminal 282, successively through the video signal processing circuit 15, control circuit 17, and video signal processing circuit 25 of the universal cord 21.

The video signal that is input to the video signal terminal 282 is signal-processed by the video signal processing unit 31, and desired image data is generated. The image data is converted to a wireless signal by the video signal transmission/reception unit 32, and is transmitted, for example, via the antenna 33 to the display device 34 and displayed. In FIG. 1, for a reason of drawing, the display device 34 is placed on the endoscope control device 27. The display device 34, however, may be disposed on other places.

The first and second transmission transformers 283 and 284 are connected to the power supply unit 30 via the control unit 29.

A lamp 35 is optically connected to the light guide connection terminal 285. The lamp lighting power unit 36 is connected to the lamp 35. The control unit 29 and power supply unit 30 are connected to the lighting power unit 36.

In a case where an operation switch (not shown) for illumination light of the operation unit 11 of the endoscope 10 is operated, an operation signal therefrom is input to the control unit 29. A driving signal, which is generated by the control unit 29 on the basis of the operation signal, is input to the lighting power unit 36. Then, the lighting power unit 36 selectively controls the driving of the lamp 35 in response to the driving signal, and causes the lamp 35 to emit illumination light to the light guide connection terminal 285.

The conduit connector receiving section 43 is provided with a water feed port 431, an air feed port 432, a sub-water feed port 433 and a suction port 434. One end of a water feed path 44 is connected to the water feed port 431. The other end of the water feed path 44 is connected to a discharge port of the water feed bottle 49 via a water feed flow amount sensor 48. A discharge port of a first pump 50 is piped and connected to a supply port of the water feed bottle 49 via a first electromagnetic valve 51.

One end of an air feed path 45 is connected to the air feed port 432. The other end of the air feed path 45 is connected to a path between the first electromagnetic valve 51 and the first pump 50 via an air feed flow amount sensor 53 and a second electromagnetic valve 52.

One end of a sub-water feed path 46 is connected to the sub-water feed port 433. The other end of the sub-water feed path 46 is connected to a discharge port of the sub-water feed bottle 55 via a sub-water feed flow amount sensor 54. A discharge port of a second pump 56 is piped and connected to a supply port of the sub-water feed bottle 55.

One end of a suction path 47 is connected to the suction port 434. The other end of the suction path 47 is piped and connected to a supply port of the suction bottle 58 via a suction flow amount sensor 57. A suction port of a third pump 59 is piped and connected to a suction port of the suction bottle 58.

The air feed flow amount sensor 53, water feed flow amount sensor 48, sub-water feed flow amount sensor 54 and suction flow amount sensor 57 are connected to the flow amount detection control unit 61. The flow amount detection control unit 61 is connected to the control unit 29 and power supply unit 30.

The first to third pumps 50, 56 and 59 and the first and second electromagnetic valves 51 and 52 are connected to the power supply unit 30 and control unit 29 via the pump/electromagnetic valve control unit 60.

The pump/electromagnetic valve control unit 60 operates, as will be described below, in accordance with the operations of the air feed operation switch 18, water feed operation switch 19, sub-water feed operation switch (not shown) and suction operation switch 20 of the operation section 11 of the endoscope 10.

When the air feed operation switch 18 of the operation section 11 of the endoscope 10 is operated, an operation signal therefrom is input to the control unit 29. A driving signal, which is generated by the control unit 29 on the basis of this operation signal, is input to the pump/electromagnetic valve control unit 60. Then, the pump/electromagnetic valve control unit 60 drives the first pump 50 in response to the driving signal, and closes the first electromagnetic valve 51 and opens the second electromagnetic valve 52. Thereby, air from the first pump 50 is supplied from the air feed path 45 to the air feed port 432 of the conduit connector receiving section 43 via the second electromagnetic valve 52. Further, the air is fed to the air feed conduit 38 of the insertion section 12 via a coupling section between the conduit connector receiving section 43 and the tube connector 42. At this time, the air feed flow amount sensor 53 detects the flow amount of passing air and outputs a detection result to the flow amount detection control unit 61.

In addition, when the water feed operation switch 19 of the operation section 11 of the endoscope 10 is operated, an operation signal therefrom is input to the control unit 29. A driving signal, which is generated by the control unit 29 on the basis of this operation signal, is input to the pump/electromagnetic valve control unit 60. Then, the pump/electromagnetic valve control unit 60 drives the first pump 50 in response to the driving signal, and opens the first electromagnetic valve 51 and closes the second electromagnetic valve 52. Thereby, water in the water feed bottle 49 is supplied from the discharge port through the water feed path 44 to the water feed port 431 of the conduit connector receiving section 43. Further, the water is fed to the water feed conduit 37 of the insertion section 12 via the coupling section between the conduit connector receiving section 43 and the tube connector 42. At this time, the water feed flow amount sensor 48 detects the flow amount of water passing through the water feed path 44, and outputs a detection result to the flow amount detection control unit 61.

When the sub-water feed operation switch (not shown) of the operation section 11 of the endoscope 10 is operated, an operation signal therefrom is input to the control unit 29. A driving signal, which is generated by the control unit 29 on the basis of this operation signal, is input to the pump/electromagnetic valve control unit 60. Then, the pump/electromagnetic valve control unit 60 drives the second pump 56 in response to the driving signal. Thereby, water in the sub-water feed bottle 55 is supplied from the discharge port thereof through the sub-water feed path 46 to the sub-water feed port 433 of the conduit connector receiving section 43. Further, the water is fed to the sub-water feed conduit 39 of the insertion section 12 via the coupling section between the conduit connector receiving section 43 and the tube connector 42. At this time, the sub-water feed flow amount sensor 54 detects the flow amount of sub-feed water passing through the sub-water feed path 46, and outputs a detection result to the flow amount detection control unit 61.

Further, when the suction operation switch 20 of the operation section 11 of the endoscope 10 is operated, an operation signal therefrom is input to the control unit 29. A driving signal, which is generated by the control unit 29 on the basis of this operation signal, is input to the pump/electromagnetic valve control unit 60. Then, the pump/electromagnetic valve control unit 60 drives the third pump 59 in response to the driving signal. Thereby, a suction force of the third pump 59 acts on the suction conduit 40 of the insertion section 12 via the suction bottle 58, suction path 47, water feed port 433 of the conduit connector receiving section 43 and the tube connector 42. Matter to be sucked, such as removed matter in the large intestine, is sucked from the suction conduit 40. At this time, the suction flow amount sensor 57 detects the flow amount of the sucked matter passing through the suction path 47, and outputs a detection result to the flow amount detection control unit 61.

The flow amount detection control unit 61 operates as follows. Specifically, if the detection signals of the air feed flow amount sensor 53, water feed flow amount sensor 44, sub-water feed flow amount sensor 54 and suction flow amount sensor 57 are input to the flow amount detection control unit 61, the flow amount detection control unit 61 calculates an air feed amount, a water feed amount including a sub-water feed amount, and a suction amount on the basis of the detection signals, and outputs information on the calculated amounts to the control unit 29. Based on the input information on the air feed amount, water feed amount and suction amount, the control unit 29 calculates a cumulative air feed amount, a cumulative water feed amount and a cumulative suction amount, and outputs the calculation result to the separate operation section 62 via the external connection connector 271 provided in the endoscope control device 27 (see FIG. 3).

The separate operation section 62 is provided such that the separate operation section 62 can remotely be disposed, for example, via a cable 621. The separate operation section 62 is provided with a current-state display section 63 and a cumulative display section 64. The current-state display section 63 displays an air feed amount, a water feed amount and a suction amount. The cumulative display section 64 displays a cumulative air feed amount, a cumulative water feed amount and a cumulative suction amount.

In addition, the separate operation section 62 is provided with an air feed switch (SW) 65, a water feed switch (SW) 66, a sub-water feed switch (SW) 67, a suction switch (SW) 68 and an external change-over switch 69. The external change-over switch 69 is a priority switch for switching between a master side and a slave side. The air feed switch 65, water feed switch 66, sub-water feed switch 67, suction switch 68 and external change-over switch 69 are connected to the pump/electromagnetic valve control unit 60 via the control unit 29.

For example, if the external change-over switch 69 is changed over to the master side, the air feed operation switch 18, water feed operation switch 19, sub-water feed operation switch (not shown) and suction operation switch 20 of the operation section 11 of the endoscope 10 are set to be operable. If the external change-over switch 69 is changed over to the slave side, the air feed switch 65, water feed switch 66, sub-water feed switch 67 and suction switch 68 of the separate operation section 62 are set to be preferentially operable.

Hence, the system of the endoscope apparatus according to this embodiment is used, for example, in the state in which an instructor of endoscopic therapeutic techniques holds the separate operation section 62 while a doctor who receives instruction operates the operation section 11 of the electronic endoscope 10. In this state, the instructor of endoscopic therapeutic techniques confirms the data displayed on the current-state display section 63 and cumulative display section 64 of the separate operation section 62, thereby determining the states of air feed, water feed and suction operations of the insertion section 12 by the doctor who receives instruction. The instructor selectively operates the external change-over switch 29 of the separate operation section 62, when necessary. If the external change-over switch 69 is changed over to the slave side, the air feed switch 65, water feed switch 66, sub-water feed switch 67 and suction switch 68 of the separate operation section 62 are switched to the state in which the operations of these switches are preferentially performed. In this manner, by selectively changing over the external change-over switch 29 of the separate operation section 62, the instruction of endoscopic therapeutic techniques can be conducted.

Furthermore, the separate operation section 62 is provided with an alarm display section 70 which constitutes alarm means. The lighting of the alarm display section 70 is controlled via the control unit 29, for example, when the control unit 29 determines that the cumulative air feed amount, cumulative water feed amount and cumulative suction amount have reached arbitrary preset values.

The present embodiment shows the structure in which the alarm display section 70 is provided on the separate operation section 62. Alternatively, the embodiment may be configured, for example, such that an alarm is displayed by flickering desired display on the current-state display section 63 and cumulative display section 64 of the separate operation section 62, or an alarm sound is produced.

The separate operation section 62 may be disposed at a proper separate position, such as a position where confirmation can be made by the doctor who operates the operation section 11 of the endoscope 10, or a position where confirmation can be made by other doctors. In this case, the doctor who operates the operation section 11 of the endoscope 10 recognizes the air feed, water feed and suction states by visually recognizing the display on the current-state display section 63 and cumulative display section 64.

Next, the operation of the present embodiment with the above-described structure is described. In the above-described structure, when the system of the endoscope apparatus of this embodiment is used, the attachment/detachment section 11a of the operation section 11 of the endoscope 10 and the connection end 12a of the insertion section 12 are coupled. Thereby, the transmission/reception coil 121 of the insertion section 12 is coupled to the transmission/reception coil 13 of the operation section 11.

Further, the scope connector 22 is attached to the scope connector receiving section 28 of the endoscope control device 27. At the same time, the tube connector 42 of the insertion section 12 is attached to the conduit connector receiving section 43 of the endoscope control device 27. At this time, the separate operation section 62 is connected via the cable 621 to the external connection connector 271 of the endoscope control device 27. The separate operation section 62 is disposed, for example, at a position where the separate operation section 62 can be visually recognized by the doctor who operates the operation section 11 of the endoscope 10.

Subsequently, the distal end portion of the insertion section 12 of the endoscope 10 is inserted into the anus of the patient A. In this state, the insertion section 12 of the endoscope 10 is further pushed in. At this time, an operation is performed to insert the insertion section 12 of the endoscope 10 into the large intestine while bend-operating the bend section 3 by the bend operation device 5 of the operation section 11 of the endoscope 10. In this case, the lamp operation section (not shown) of the operation section 11 of the endoscope 10 is operated to drive the lamp 35 via the lamp lighting power unit 36, thereby causing the lamp 35 to emit illumination light. The illumination light is guided to the light guide 26A of the insertion section 12 via the light guide 26B within the universal cord 21. The illumination light that is propagated to the distal end of the light guide 26A is emitted to the outside via the illumination lens disposed in the distal end hard section 4. The illumination light is radiated in the large intestine, thus illuminating the inside of the large intestine.

An observation image of the endoscope 10 (an image of the inside of the large intestine), which is incident through the observation lens disposed in the distal end hard section 4, is captured by the imaging device 123 such as a CCD. The observation image of the endoscope 10, which is captured by the imaging device 123, is converted to an electric signal. An output signal of the image data from the imaging device 123 is input to the video signal processing unit 31 via an electric signal line within the insertion section 12 and operation section 11 of the endoscope 10 and an electric signal line within the universal cord 21. The electric signal, which is signal-processed in the video signal processing unit 31, is transmitted from the antenna 33 to the display device 34 via the video signal transmission/reception unit 32 and is input to the display device 34. Thus, the observation image of the endoscope 10 is displayed on the display screen 34a for endoscopic images.

In the case where air is fed into the large intestine from the air feed conduit 38 when the insertion section 12 is inserted, the air feed operation switch 18 of the operation section 11 is operated. When the air feed operation switch 18 is operated, the first pump 50 of the endoscope control device 27 is driven as described above, and the first electromagnetic valve 51 is closed and the second electromagnetic valve 52 is opened. Thereby, air is supplied into the air feed conduit 38, and the air is fed from the air feed conduit 38 of the insertion section 12 that is inserted in the large intestine.

Then, in the case where water is fed to clean the distal-end lens of the insertion section 12, the water feed operation switch 19 of the operation section 11 is operated. Water in the water feed bottle 49 is fed to the water feed conduit 37, as described above, and the water is fed to the distal-end lens from the water feed conduit 37, thereby carrying out cleaning.

In the case where the inside of the large intestine is cleaned, the sub-water feed operation switch (not shown) of the operation section 11 is operated. The second pump 56 is driven, and water in the sub-water feed bottle 55 is fed into the sub-water feed conduit 39 of the insertion section 12, thereby carrying out cleaning of the inside of the large intestine.

In the case of sucking treated matter, etc. in the large intestine, the suction operation switch 20 of the operation section 11 is operated. Then, as described above, the third pump 59 is driven to effect suction. At this time, the suction force of the third pump 59 is caused to act on the suction conduit 40 of the insertion section 12 via the suction bottle 58, suction path 47, suction port 434 of the conduit connector receiving section 43 and the tube connector 42. Thus, matter to be sucked, such as removed matter, in the large intestine is sucked by the suction force from the suction conduit 40, and is put in the suction bottle 58.

At this time, the detection signals of the air feed flow amount sensor 53, water feed flow amount sensor 48, sub-water feed flow amount sensor 54 and suction flow amount sensor 57 are input to the flow amount detection control unit 61 of the endoscope control device 27. On the basis of these detection signals, the air feed amount, the water feed amount including the sub-water feed amount and the suction amount are calculated and are output to the control unit 29. Based on the input information on the air feed amount, water feed amount and suction amount, the control unit 29 calculates the cumulative air feed amount, cumulative water feed amount and cumulative suction amount, and outputs them to the separate operation section 62. Further, an alarm signal is output to the separate operation section 62 when the cumulative air feed amount, cumulative water feed amount and cumulative suction amount have reached preset values.

In the separate operation section 62, the current-state display section 63 displays the air feed amount, water feed amount and suction amount. Further, the cumulative display section 64 displays the cumulative air feed amount, cumulative water feed amount and cumulative suction amount. When the alarm signal is input, the alarm display section 70 is lighted to give an alarm. The doctor who operates the operation section 11 of the endoscope 10 visually recognizes the display on the current-state display section 63 and cumulative display section 64 of the separate operation section 62. Thereby, the doctor understands the air feed, water feed and suction states, and performs therapeutic treatment, for instance. In addition, when the lighting of the alarm display section 70 is confirmed, the air feed, water feed and suction are temporarily stopped. Thereafter, when safety is confirmed, the air feed, water feed and suction operations are performed once again.

The separate operation section 62 is held, for example, by the hand of a second doctor who is different from a first doctor who operates the operation section 11 of the endoscope 10, and is used to give instruction of endoscopic therapeutic techniques to the first doctor who operates the operation section 11 of the endoscope 10. In the case of this mode of use, the second doctor can determine whether the values are proper or not, on the basis of the display values on the current-state display section 63 and cumulative display section 64 of the separate operation section 62. Alternatively, in the state in which the alarm display section 70 is lighted, the external change-over switch 69 is changed over to the slave side. Thereby, the air feed switch 65, water feed switch 66, sub-water feed switch 67 and suction switch 68 are selectively operated, and the operations of air feed, water feed and suction from the insertion section 12 can be stopped.

In addition, by the change-over operation of the external change-over switch 69 to the slave side, the operation section 11 of the endoscope 10 is locked and switched to the non-operable state, and the separate operation section 62 is switched to the preferentially operable state. Thereby, the first doctor, who operates the operation section 11 of the endoscope 10, can somesthetically learn the timing of the air feed, water feed and suction operations.

In an alternative mode of use, the first doctor who receives instruction may hold the separate operation section 62 by the hand, and the second doctor who gives instruction may operate the operation section 11 of the endoscope 10. In this case, too, the second doctor observes the current-state display section 63 and cumulative display section 64 of the separate operation section 62, and can thus numerically learn therapeutic techniques.

The separate operation section 62 may be used by one person or by a plurality of persons. Thereby, the timing of the air feed, water feed and suction operations can numerically be understood.

The following advantageous effects can be obtained by the above-described structure. Specifically, in the system of the endoscope apparatus of the present embodiment, the separate operation section 62, which can be disposed separately from the operation section 11 of the endoscope 10, is provided. This separate operation section 62 is provided with the current-state display section 63, cumulative display section 64, air feed switch 65, water feed switch 66, sub-water feed switch 67 and suction switch 68. Thereby, if air feed, water feed and suction are performed by the operation section 11, the air feed amount, water feed amount and suction amount can be detected and displayed on the current-state display section 63 and cumulative display section 64 of the separate operation section 62. The air feed amount, water feed amount and suction amount can be understood by confirming the display data on the current-state display section 63 and cumulative display section 64 of the separate operation section 62. Further, by the operation of changing over the external change-over switch 69 of the separate operation section 62 to the slave side, the air feed switch 65, water feed switch 66, sub-water feed switch 67 and suction switch 68 on the separate operation section 62 side can selectively be operated in place of the air feed, water feed and suction operations of the operation section 11. Thereby, the operation of the endoscope 10 can be controlled.

According to the embodiment, the air feed, water feed and suction operations with high reliability and high precision can simply and easily be performed in the insertion operation of the insertion section 12, and the handling operability of operations, including the insertion operation of the insertion section 12, can be improved.

In the above-described embodiment, the separate operation section 62 is connected to the endoscope control device 27 over the cable 621 so that the separate operation section 62 may be remotely disposed. Alternatively, for example, the operation section 62 and the endoscope control device 27 may be wirelessly connected by a wireless structure. In this case, there is no restriction to the position of the separate operation section 62, and the handling operability can further be improved.

In the above-described embodiment, the invention is applied to the endoscope system in which the operation section 11 of the endoscope 10 and the insertion section 12 are separable. Alternatively, the invention is applicable to an endoscope system in which an operation section of an endoscope body and an endoscope insertion section are integrally coupled as one unit, and the same advantageous effects as described above can be obtained.

Besides, in the above-described embodiment, the invention is applied to the case in which the object of an inspection is the large intestine. Alternatively, the invention is applicable to therapeutic treatments including an inspection of other organs, etc., and the same advantageous effects as described above can be obtained.

The present invention is not limited to the above-described embodiment. At the stage of practicing the invention, various modifications may be made. Further, the embodiment includes various inventions at various stages, and various inventions may be derived by properly combining structural elements disclosed in the embodiment.

## Claims

1. An endoscope apparatus comprising:
an endoscope (10) including an insertion section (12) which is adapted to be inserted in a lumen that is an object of an inspection, and an operation section (11) having an operation switch (18, 19, 20) for controlling an operation element adapted for performing an operation of the insertion section (12);
detection means (61) adapted for detecting an operation state of the operation element; and
a separate operation section (62) disposed separately from the endoscope (10) comprising a separate operation section switch (65-68) adapted to operate and control the operation of the operation element
**characterized in that** the separate operation section (62) includes:
a display section (63, 64, 70) adapted to display the operation state of the operation element detected by the detection means (61), and
a switching means (69) that is operable for switching control of the operation element between a first state in which the operation switch (18-20) of the operation section (11) controls the operation element, and a second state in which the operation element is controlled by the separate operation section switch (65-68) of the separate operation section (62) and the operation section (11) is locked and switched to a non-operable state.

2. The endoscope apparatus according to claim 1, **characterized in that**
the operation switch (18, 19, 20) of the operation section (11) includes an air feed operation switch (18), a water feed operation switch (19), and a suction operation switch (20) operable to control the air feed mechanism, the water feed mechanism and the suction mechanism, respectively,
the operation element includes:
an air feed mechanism adapted to feed air to an air feed conduit (38) and to feed the air into the object of the inspection;
a water feed mechanism adapted to feed water to a water feed conduit (37) and to feed the water into the object of the inspection; and
a suction mechanism having a suction conduit (40) adapted to suck, via the suction conduit (40), matter to be sucked in the object of the inspection,
the detection means (61) detects an air feed amount of the air that is fed from the air feed mechanism, a water feed amount of the water that is fed from the water feed mechanism, and a suction amount of the matter sucked by the suction mechanism, and
the display section (63, 64, 70) of the separate operation section (62) is adapted to display the air feed amount, the water feed amount and the suction amount which are detected by the detection means (62), and
the separate operation section, switch (65-68) comprises a separate operation section air feed operation switch (66), a separate operation section water feed operation switch (67), and a separate operation section suction operation switch (68) which are adapted to be operable to control the air feed mechanism, the water feed mechanism and the suction mechanism, respectively.

3. The endoscope apparatus according to claim 1, **characterized in that** the separate operation section (62) includes alarm means (70) adapted for giving an alarm when the air feed amount, the water feed amount and the suction amount, which are detected by the detection means (61), have reached arbitrary preset values.

4. The endoscope apparatus according to claim 3, **characterized in that** the alarm means (70) is adapted to flicker a display of the air feed amount, the water feed amount and the suction amount on the display section (63, 64, 70).

5. The endoscope apparatus according to claim 1, **characterized in that** the alarm means (70) is adapted to produce an alarm sound.

6. The endoscope apparatus according to claim 1, **characterized in that** the separate operation section (62) is adapted to be wirelessly connected to the detection means (61).

## Patentansprüche

1. Endoskopgerät, das umfasst:
ein Endoskop (10), das einen Einführabschnitt (12), der dazu eingerichtet ist, in ein Lumen eingeführt zu werden, das ein Untersuchungsobjekt ist, und einen Betätigungsabschnitt (11) umfasst, der einen Betätigungsschalter (18, 19, 20) zum Steuern eines Betätigungselements hat, welches dazu eingerichtet ist, eine Betätigung des Einführabschnitts (12) auszuführen;
ein Erfassungsmittel (61), das dazu eingerichtet ist, einen Betätigungszustand des Betätigungselements zu erfassen; und
einen separaten Betätigungsabschnitt (62), der separat von dem Endoskop (10) angeordnet ist und einen separaten Betätigungsabschnittsschalter (65-68) umfasst, der dazu eingerichtet ist, die Betätigung des Betätigungselements zu betätigen und zu steuern,
**dadurch gekennzeichnet, dass** der separate Betätigungsabschnitt (62) umfasst:
einen Anzeigeabschnitt (63, 64, 70), der dazu eingerichtet ist, den durch das Erfassungsmittel (61) erfassten Betätigungszustand des Betätigungselements anzuzeigen, und
ein Schaltmittel (69), das betätigbar ist, um eine Steuerung des Betätigungselements zwischen einem ersten Zustand, in dem der Betätigungsschalter (18-20) des Betätigungsabschnitts (11) das Betätigungselement steuert, und einem zweiten Zustand umzuschalten, in dem das Betätigungselement durch den separaten Betätigungsabschnittsschalter (65-68) des separaten Betätigungsabschnitts (62) gesteuert wird und der Betätigungsabschnitt (11) gesperrt und in einen nicht betätigbaren Zustand geschaltet ist.

2. Endoskopgerät gemäß Anspruch 1, **dadurch gekennzeichnet, dass**
der Betätigungsschalter (18, 19, 20) des Betätigungsabschnitts (11) einen Luftzufuhrbetätigungsschalter (18), einen Wasserzufuhrbetätigungsschalter (19) und einen Saugbetätigungsschalter (20) umfasst, die betätigbar sind, um jeweils den Luftzufuhrmechanismus, den Wasserzufuhrmechanismus und den Saugmechanismus zu steuern,
wobei das Betätigungselement umfasst:
einen Luftzufuhrmechanismus, der dazu eingerichtet ist, einer Luftzufuhrleitung (38) Luft zuzuführen und die Luft in das Untersuchungsobjekt zuzuführen;
einen Wasserzufuhrmechanismus, der dazu eingerichtet ist, einer Wasserzufuhrleitung (37) Wasser zuzuführen und das Wasser in das Untersuchungsobjekt zuzuführen; und
einen Saugmechanismus mit einer Saugleitung (40), die dazu eingerichtet ist, einen zu saugenden Stoff über die Saugleitung in das Untersuchungsobjekt zu saugen,
wobei das Erfassungsmittel (61) einen Luftzufuhrbetrag der Luft, die durch den Luftzufuhrmechanismus zugeführt wird, einen Wasserzufuhrbetrag des Wassers, das durch den Wassermechanismus zugeführt wird, und einen Saugbetrag des durch den Saugmechanismus gesaugten Stoffs erfasst, und
der Anzeigeabschnitt (63, 64, 70) des separaten Betätigungsabschnitts (62) dazu eingerichtet ist, den Luftzufuhrbetrag, den Wasserzufuhrbetrag und den Saugbetrag, die durch das Erfassungsmittel (62) erfasst werden, anzuzeigen, und
der separate Betätigungsabschnittsschalter (65- 68) einen separaten Betätigungsabschnittsluftzufuhrbetätigungsschalter (66), einen separaten Betätigungsabschnittswasserzufuhrbetätigungsschalter (67) und einen separaten Betätigungsabschnittssaugbetätigungsschalter (68) umfasst, die dazu eingerichtet sind, betätigbar zu sein, um jeweils den Luftzufuhrmechanismus, den Wasserzufuhrmechanismus und den Saugmechanismus zu steuern.

3. Endoskopgerät gemäß Anspruch 1, **dadurch gekennzeichnet, dass** der separate Betätigungsabschnitt (62) ein Alarmmittel (70) umfasst, das dazu eingerichtet ist, einen Alarm zu geben, wenn der Luftzufuhrbetrag, der Wasserzufuhrbetrag und der Saugbetrag, die durch das Erfassungsmittel erfasst werden, beliebige voreingestellt Werte erreicht haben.

4. Endoskopgerät gemäß Anspruch 3, **dadurch gekennzeichnet, dass** das Alarmmittel (70) dazu eingerichtet ist, eine Anzeige des Luftzufuhrbetrags, des Wasserzufuhrbetrags und des Saugbetrags auf dem Anzeigeabschnitt (63, 64, 70) blinken zu lassen.

5. Endoskopgerät gemäß Anspruch 1, **dadurch gekennzeichnet, dass** das Alarmmittel (70) dazu eingerichtet ist, einen Alarmton zu erzeugen.

6. Endoskopgerät gemäß Anspruch 1, **dadurch gekennzeichnet, dass** der separate Betätigungsabschnitt (62) dazu eingerichtet ist, drahtlos mit dem Erfassungsmittel (61) verbunden zu sein.

## Revendications

1. Appareil d'endoscope comprenant :
un endoscope (10) comprenant une section d'insertion (12) qui est adaptée pour être insérée dans une lumière qui est un objet d'une inspection, et une section d'opération (11) comportant un commutateur d'opération (18, 19, 20) destiné à commander un élément d'actionnement adapté pour réaliser un actionnement de la section d'insertion (12) ;
un moyen de détection (61) adapté pour détecter un état de fonctionnement de l'élément d'actionnement ; et
une section d'opération séparée (62) disposée séparément de l'endoscope (10) comprenant un commutateur de section d'opération séparée (65-68) adapté pour fonctionner et commander le fonctionnement de l'élément d'actionnement
**caractérisé en ce que** la section d'opération séparée (62) comprend :
une section d'affichage (63, 64, 70) adaptée pour afficher l'état de fonctionnement de l'élément d'actionnement détecté par le moyen de détection (61), et
un moyen de commutation (69) qui est opérable pour commuter la commande de l'élément d'actionnement entre un premier état dans lequel le commutateur d'opération (18 à 20) de la section d'opération (11) commande l'élément d'actionnement, et un deuxième état dans lequel l'élément d'actionnement est commandé par le commutateur de section d'opération séparée (65-68) de la section d'opération séparée (62) et la section d'opération (11) est bloquée et commutée vers un état non opérable.

2. Appareil d'endoscope selon la revendication 1, **caractérisé en ce que**
le commutateur d'opération (18, 19, 20) de la section d'opération (11) comprend un commutateur d'opération d'alimentation en air (18), un commutateur d'opération d'alimentation en eau (19) et un commutateur d'opération d'aspiration (20) opérables pour commander le mécanisme d'alimentation en air, le mécanisme d'alimentation en eau et le mécanisme d'aspiration, respectivement,
l'élément d'actionnement comprend :
un mécanisme d'alimentation en air adapté pour délivrer de l'air vers un conduit d'alimentation en air (38) et pour délivrer l'air dans l'objet de l'inspection ;
un mécanisme d'alimentation en eau adapté pour délivrer de l'eau vers un conduit d'alimentation en eau (37) et pour délivrer l'eau dans l'objet de l'inspection ; et
un mécanisme d'aspiration comportant un conduit d'aspiration (40) adapté pour aspirer, via le conduit d'aspiration (40), la matière devant être aspirée dans l'objet de l'inspection,
le moyen de détection (61) détecte une quantité d'alimentation en air de l'air qui est délivré depuis le mécanisme d'alimentation en air, une quantité d'alimentation en eau de l'eau qui est délivrée depuis le mécanisme d'alimentation en eau, et une quantité d'aspiration de la matière aspirée par le mécanisme d'aspiration, et
la section d'affichage (63, 64, 70) de la section d'opération séparée (62) est adaptée pour afficher la quantité d'alimentation en air, la quantité d'alimentation en eau et la quantité d'aspiration qui sont détectées par le moyen de détection (62), et
le commutateur de section d'opération séparée (65-68) comprend un commutateur (66) d'opération d'alimentation en air de section d'opération séparée, un commutateur (67) d'opération d'alimentation en eau de section d'opération séparée, et un commutateur (68) d'opération d'aspiration de section d'opération séparée qui sont adaptés pour être opérables pour commander le mécanisme d'alimentation en air, le mécanisme d'alimentation en eau et le mécanisme d'aspiration, respectivement.

3. Appareil d'endoscope selon la revendication 1, **caractérisé en ce que** la section d'opération séparée (60) comprend un moyen d'alerte (70) adapté pour délivrer une alerte lorsque la quantité d'alimentation en air, la quantité d'alimentation en eau et la quantité d'aspiration, qui sont détectées par le moyen de détection (61), ont atteint des valeurs arbitraires préétablies.

4. Appareil d'endoscope selon la revendication 3, **caractérisé en ce que** le moyen d'alerte (70) est adapté pour faire vaciller un affichage de la quantité d'alimentation en air, de la quantité d'alimentation en eau et de la quantité d'aspiration sur la section d'affichage (63, 64, 70).

5. Appareil d'endoscope selon la revendication 1, **caractérisé en ce que** le moyen d'alerte (70) est adapté pour produire une alerte sonore.

6. Appareil d'endoscope selon la revendication 1, **caractérisé en ce que** la section d'opération séparée (62) est adaptée pour être connectée sans fil au moyen de détection (61).
